(19) Europäisches Patentamt

European Patent Office

Office européen des brevets

(11) **EP 1 690 519 A1**

(12) **EUROPEAN PATENT APPLICATION**
published in accordance with Art. 158(3) EPC

(43) Date of publication:
**16.08.2006 Bulletin 2006/33**

(51) Int Cl.:
**A61K** /(2006.01)

(21) Application number: **04817429.6**

(22) Date of filing: **04.11.2004**

(86) International application number:
**PCT/JP2004/016333**

(87) International publication number:
**WO 2005/041995 (12.05.2005 Gazette 2005/19)**

(84) Designated Contracting States:
**AT BE BG CH CY CZ DE DK EE ES FI FR GB GR HU IE IS IT LI LU MC NL PL PT RO SE SI SK TR**

(30) Priority: **04.11.2003 JP 2003374837**

(71) Applicant: **Meiji Dairies Corporation
Koto-ku,
Tokyo 136-8908 (JP)**

(72) Inventors:
• **TSUBOI, H.
Meiji Dairies Corp. Funct. Res. Inst.
Kanagawa 2500862 (JP)**
• **IKEGAMI, S.
Meiji Dairies Corp. Funct. Res. Inst.
Kanagawa 2500862 (JP)**

• **JI, Zai-si
Meiji Dairies Corp. Funct. Res. Inst.
Kanagawa 2500862 (JP)**
• **ITOU, H.
Meiji Dairies Corp. Funct. Res. Inst.
Kanagawa 2500862 (JP)**
• **ODA, M.
Meiji Dairies Corp. Food Func. Res. Inst.
Kanagawa 2500862 (JP)**
• **SHIN, K.
Meiji Dairies Corp. Food Func. Res. Inst.
Kanagawa 2500862 (JP)**

(74) Representative: **Grünecker, Kinkeldey,
Stockmair & Schwanhäusser
Anwaltssozietät
Maximilianstrasse 58
80538 München (DE)**

(54) **ALPHA-GLUCOSIDASE ACTIVITY INHIBITOR**

(57)    The present inventors focused on the lotus plant and prepared lotus extracts. When α-glucosidase and its substrate sucrose were reacted with the lotus extracts to examine the effect of adding the extracts on the enzymatic reaction, the lotus extracts were found to have α-glucosidase inhibitory activity. Furthermore, when the lotus extract and sucrose were administered to mice and their blood glucose levels were measured, the lotus extracts were found to suppress blood glucose levels. More specifically, the present inventors discovered that the lotus extracts have α-glucosidase inhibitory activity and can be used to control blood glucose levels.

**Description**

Technical Field

The present invention relates to uses of α-glucosidase activity inhibitors that comprise plant preparations.

Background Art

Patients with diabetes, which is referred to as a lifestyle-related disease, are on the increase. In Japan, an estimated 6,900,000 people are "strongly suspected of having diabetes", and about 13,700,000 people including "those in which the possibility of having diabetes cannot be ruled out" (1997 Diabetes Survey by the Ministry of Health, Labour and Welfare). Accordingly, options for diabetes improvement are being widely called for. Diabetes is characterized by chronic high blood glucose levels due to a reduced supply of insulin or ineffective insulin, and it is one of a group of diseases that accompany various metabolic abnormalities. Depending on the degree of metabolic abnormality a range of pathologies are shown, from symptomless conditions to ketoacidosis or coma. When a metabolic abnormality continues over a long period, characteristic complications tend to develop in the retinas, kidneys and nerves, and arteriosclerosis is also accelerated. Diabetes is treated by controlling blood glucose levels using diet, exercise and drug therapies.

Oral agents for diabetes include sulfonylurea (SU) agents, biguanide (BG) agents, insulin resistance agents, and α-glucosidase inhibitors. Of these, α-glucosidase inhibitors are agents that competitively inhibit carbohydrate hydrolases such as maltase and sucrase, present in the small intestinal mucous microcilia. They also delay the absorption of carbohydrates from the intestinal tract. α-Glucosidase inhibitors such as acarbose and voglibose are already in clinical use. α-Glucosidase inhibitors have the effect of suppressing marked increases in postprandial blood glucose level and reducing the range of diurnal variation in blood glucose. In addition, α-glucosidase inhibitors are thought to be applicable to insulin-dependent diabetes patients (Journal of Nippon Medical School, 1999, 66:3). Acarbose was discovered from the culture medium of mycobacterium *Actinoplanes* strain SE50, and voglibose was discovered from the culture medium of mycobacterium *Streptomyces hygroscopicus sub sp. limoneus.*

So far there have been reports that substances with α-glucosidase inhibitory activity exist in plants (Japanese Patent Application Kokai Publication No. (JP-A) 2003-81858, and JP-A 2000-229875).

[Patent Document 1] JP-A 2003-81858
[Patent Document 2] JP-A 2000-229875
[Patent Document 3] JP-A H8-198769
[Non-Patent Document 1] Journal of Nippon Medical School, 1999, 66:3, p.195-198

Disclosure of the Invention

Problems to be Solved by the Invention

The present invention was achieved in view of the above circumstances. An objective of the present invention is to provide plant-derived novel α-glucosidase inhibitory substances, and therapeutic or preventive agents for diabetes which use the α-glucosidase inhibitory substances.

Means to Solve the Problems

The present inventors conducted dedicated research to solve the above-mentioned problems, and to discover in plants substances with α-glucosidase inhibitory activity.

The present inventors focused on the lotus (*Nelumbonaceae Nelumbo*) plant, which is a perennial *Nelumbonaceae* herb. Lotus roots are used for food, and lotus seeds and leaves are widely used in formulating Chinese herbal medicines, and in health foods. Lotus leaves are well known to have obesity-improving effects (JP-A H8-198769), but their effect in suppressing increases in blood glucose level is not known.

The present inventors produced lotus extracts, reacted these with α-glucosidase and its substrate sucrose, and measured the amount of glucose produced. The effect of adding a lotus extract on the enzymatic reaction was calculated using measured glucose levels, clarifying that the lotus extracts had α-glucosidase inhibitory activity. When the present inventors administered the lotus extracts and sucrose to mice, the lotus extracts were found to suppress the blood glucose levels of the mice. Furthermore, the present inventors performed a glucose tolerance test on humans who were given the lotus extracts, and confirmed that the lotus extracts suppressed increases in blood glucose level. More specifically, the present inventors showed that the lotus extracts have α-glucosidase inhibitory activity and can be used to control blood glucose levels.

Specifically, the present invention provides:

(1) an α-glucosidase activity inhibitor comprising a *Nelumbonaceae* plant preparation;
(2) a pharmaceutical composition for treating or preventing diabetes, wherein the composition comprises the α-glucosidase activity inhibitor of (1); and
(3) a food composition for treating or preventing diabetes, wherein the composition comprises the α-glucosidase activity inhibitor of (1).

Brief Description of the Drawings

Fig. 1 is a graph showing the blood glucose-suppressing effect of lotus leaf extract in sucrose-loaded mice. Mean ± S.D. (n=4).
Fig. 2 shows the glucose tolerance test results obtained from the lotus leaf extract-administered group (humans) and the control group (humans). Blood glucose levels at certain times after glucose loading are shown as relative glucose levels (%) with respect to the glucose level before glucose loading. Mean ± S.D. Group S (placebo), n=30; Group T (dried lotus leaf extract 1 g/day), n=34; and Group R (dried lotus leaf extract 2 g/day), n=31.

Best Mode for Carrying Out the Invention

The present invention provides α-glucosidase activity inhibitors that comprise *Nelumbonaceae* plant preparations. As described in the Examples, the lotus preparations were found to have an α-glucosidase inhibitory effect. The present invention can be used for any application, as long as the application is expected to inhibit the activity of an α-glucosidase (maltase, sucrase, etc.) in degrading disaccharides to monosaccharides. In particular, administration of α-glucosidase activity inhibitors to animals inhibits the degradation of disaccharides to monosaccharides by maltase and such in the small intestine, and delays the uptake of carbohydrates in the intestinal tract. As a result, such administrations are expected to suppress increases in blood glucose level. The effect of the α-glucosidase activity inhibitors of the present invention in suppressing increases in blood glucose levels has been demonstrated in animal tests, as described in the Examples.

*Nelumbonaceae* plants include lotus (*Nelumbo nucifera*) and *Nelumbo lutea.* The α-glucosidase activity inhibitory effect is believed to be not only present in lotus preparations, but also in preparations from other *Nelumbonaceae* plants and closely related *Nymphaea, Euryale, Nuphar,* and *Brasenia.* Therefore, substances with an α-glucosidase activity inhibitory effect can be obtained from these plants. In the present invention, plant preparations refer to substances obtained by processing an entire plant or a part of a plant. For example, plant extracts, plant juices, plant essences, dried plants and crushed plants are included in the plant preparations of the present invention. The parts of a plant used to produce a preparation are not limited, so long as the preparation obtained from those parts has an α-glucosidase activity inhibitory effect. In the case of lotus, leaves are an example of a part that is suitable for use. Methods for producing plant preparations are not particularly limited so long as an α-glucosidase activity inhibitory effect can be derived. Extraction using a solvent or methods that powderize plants may be used. Examples of solvents which can be used for such extractions include water (which may comprise acids, bases, salts and surfactants), and organic solvents generally used in extractions, such as alcohols including methanol, ethanol, isopropanol and butanol, and ethyl acetate, acetone, tetrahydrofuran, chloroform, dichloromethane, and acetonitrile. These solvents may be used alone or in combination as mixed solvents. Supercritical fluids may also be used. Water is an example of a suitable solvent. More specifically, preparation may be by the methods in the Examples.

Whether a substance is an α-glucosidase activity inhibitor or not can be evaluated by adding a test substance to an α-glucosidase and its substrate disaccharide, examining the enzymatic reaction, and determining the inhibition ratio of the enzymatic reaction. To determine inhibition ratio, the amount of product produced from the substrate as a result of an enzymatic reaction is measured in the presence and absence of a test substance, and the difference between these measurements is divided by the value measured in the absence of the test substance. The amount of enzymatic reaction product formed from a substrate can be measured by various methods commonly known to those skilled in the art. For example, glucose can be measured by a method suitably selected from absorbance measurement methods, electrode methods, glucose oxidase methods, HK/G-6-PD methods, liquid chromatographic methods, and such.

The present invention provides therapeutic or preventive pharmaceutical agents for diabetes that comprise an above-mentioned α-glucosidase activity inhibitor. The effect of an α-glucosidase activity inhibitor in suppressing increases in blood glucose level has been proven in humans and mice, as described above. Therefore the α-glucosidase activity inhibitors can obviously be used as therapeutic or preventive pharmaceutical agents for diabetes in humans and other mammals, including rodents. Therapy or prevention of diabetes in the present invention includes treatments for diabetes patients, prevention of diabetes development in so-called borderline groups of those who may develop diabetes, as well as uses aimed at suppressing increases in postprandial blood glucose in individuals, regardless of whether or not they are diabetes patients.

The therapeutic or preventive pharmaceutical compositions for diabetes of the present invention are prepared as conventional pharmaceutical formulations. For example, the α-glucosidase activity inhibitors are formulated as pharmaceutical compositions obtained by mixing with pharmaceutically acceptable carriers (excipients, binders, disintegrators, corrigents, flavors, emulsifiers, diluents, solubilizers, etc.). Alternatively, they are formulated as formulations such as tablets, pills, powders, granules, capsules, troches, syrups, liquid agents, emulsions, suspensions, or injections, in a form suitable for oral or parenteral administration. Excipients include lactose, corn starch, saccharose, glucose, sorbitol, and plasma cellulose. Binders include polyvinyl gum arabia, tragacanth, gelatin, shellac, hydroxypropyl cellulose, hydroxypropyl starch, and polyvinylpyrrolidone.

As described in the Examples, whether or not an agent is effective for diabetes can be confirmed by, for example: administering an agent to test animals at feeding time; monitoring the blood glucose level over time after feeding; and observing the suppressive effect of the agent on blood glucose level. Alternatively, the efficacy of an agent can be confirmed by: administering an agent to animals that have developed diabetes; and then measuring blood glucose level in the test animals. Blood glucose at any given time before or after agent administration is then compared between a group to which a test substance was administered, and a control group. If the blood glucose level of the agent-administered group is less than that of the control group, that agent is effective against diabetes.

Further, the present invention provides therapeutic or preventive food compositions for diabetes, which comprise α-glucosidase activity inhibitors that comprise an above-mentioned *Nelumbonaceae* plant preparation. *Nelumbonaceae* plants are very safe, have been conventionally taken as food, and can be processed into food compositions. *Nelumbonaceae* plant preparations suppress increases in blood glucose level and promote the gradual absorption of sugars. The food compositions of the present invention are of course useful for treating diabetes patients; however, in addition to diabetes patients, so-called borderline groups and pre-diabetes patients who may be concerned about their blood glucose can also take the food compositions of the present invention to effectively control their blood glucose levels without using pharmaceutical agents, making it possible to prevent diabetes and thus making the food compositions of the present invention particularly useful.

Examples of the food compositions of the present invention include: foods for diabetes; foods for special uses; foods for specified health uses; functional foods that can claim to suppress blood glucose level increases, suppress postprandial blood glucose level increases, and lower blood glucose levels; foods with nutrient function claims; health foods; nutritional supplement foods; and enteral nutritional foods. However, the food compositions of the present invention are not limited thereto so long as they are used for the purpose of preventing or treating diabetes, or for suppressing increases in blood glucose level. The daily doses or intakes are not particularly limited, so long as they are within a range that can be taken safely and effectively. An example of a daily intake for humans would be 0.01 g/day to 100 g/day, and preferably 0.1 g/day to 10 g/day.

The methods for producing such compositions are techniques well known and commonly used among those skilled in the art. Specifically, the α-glucosidase activity inhibitors of the present invention can be mixed with compounds acceptable in terms of food hygiene for processing into foods such as foods for diabetes, foods for special uses, foods for specified health uses, functional foods that can claim to lower blood glucose level, foods with nutrient function claims, health foods, nutritional supplement foods, and enteral nutritional foods. The α-glucosidase activity inhibitors of the present invention can be added to existing foods. For example, compounds such as stabilizers, preservatives, coloring agents, flavors, and vitamins may be in the form of granules, powders, capsules, liquids, creams, and beverages, suitable for compositions. All prior art references cited herein are incorporated by reference into this description.

[Examples]

Hereinbelow, the present invention will be specifically described with reference to Examples, but it is not to be construed as being limited thereto.

[Example 1] Preparation of *Nelumbonaceae* plant extracts

Ten liters of water was added to 1 kg of dried lotus leaves. The mixture was adjusted to pH 6.0 and left to stand at room temperature for 30 minutes. Extraction was then carried out by boiling this mixture under reduced pressure at 90°C for one hour. Filtrate 1 was separated from the residue. Ten equivalents of water was then added to the residue. Then, extraction was carried out again by boiling this mixture under reduced pressure at 90°C for one hour, whereupon filtrate 2 and residue were obtained by separation. Filtrate 1 and filtrate 2 were combined, concentrated by heating under reduced pressure to a specific gravity of 1.1, and then dried using a spray drier to obtain 100 g of dried powder.

[Example 2] Preparation of α-glucosidase enzyme solution

A frozen tissue section of a rat small intestine was further firmed with liquid nitrogen, then finely ground. Five weight equivalents of 50 mM mannitol / 2 mM Tris hydrochloride buffer was then added to this material. The mixture was homogenized for two minutes under ice-cold conditions. 1 M calcium chloride solution was added to the resulting homogenates to a final concentration of 10 mM, and was further stirred at 4°C for 20 minutes. The mixture was centrifuged at 3,000 rpm for 30 minutes to remove the supernatant. This supernatant was further centrifuged at 15,000 rpm for 30 minutes at 4°C, and the precipitate was collected. This precipitate was washed twice with 10 mM maleic acid/sodium hydroxide buffer (pH5.8), and finally suspended in 10 mL of 10 mM maleic acid/sodium hydroxide buffer to obtain an α-glucosidase enzyme solution.

[Example 3] α-glucosidase inhibition reaction test

10 μL of the α-glucosidase enzyme solution, 10 μL of a test substance (various concentrations of lotus leaf extract solution), 20 μL of substrate solution (20 mg/mL sucrose and 20 mM phosphate buffer), and 10 μL of 20 mM phosphate-buffered saline were mixed and incubated at 37°C for 40 minutes. After the reaction was completed, the amount of glucose produced was measured using Glucose CII-Test Wako (Wako Pure Chemical). Since the color of the sample solution may affect the absorbance for determining the amount of glucose, a blank test (in which each test substance was added to the mixture after the enzymatic reaction) was carried out and blank values were obtained. The glucose values were corrected using the blank values to calculate the inhibition ratio, according to the following formula:

Inhibition ratio (%) =

(glucose concentration when the test substance is added after the enzymatic reaction – glucose concentration when the test substance is added from the beginning) /

glucose concentration when the test substance is added after the enzymatic reaction x 100

The results are shown in Table 1. Dried lotus leaf extract at a final concentration of 4 mg/mL inhibited α-glucosidase activity by approximately 50% when sucrose was the substrate. These results proved that the blood glucose increase after carbohydrate intake is suppressed because the active ingredient of the present invention inhibits the α-glucosidase activity required for digestion and absorption of carbohydrates.

Table 1

| Test substance | A (mg/dL) | B (mg/dL) | A−B (mg/dL) | Inhibition ratio (%) |
|---|---|---|---|---|
| Water | 72.080 | 0.280 | 71.800 | 0.00 |
| Lotus leaf extract 2mg/mL | 54.480 | 9.480 | 45.000 | 37.33 |
| Lotus leaf extract 4mg/mL | 50.280 | 13.880 | 36.400 | 49.30 |
| Lotus leaf extract 8mg/mL | 43.880 | 15.680 | 28.200 | 60.72 |
| Lotus leaf extract 12mg/mL | 39.280 | 18.280 | 21.000 | 70.75 |
| Lotus leaf extract 16mg/mL | 36.480 | 20.680 | 15.800 | 77.99 |
| Lotus leaf extract 20mg/mL | 32.680 | 21.280 | 11.400 | 84.12 |

A: glucose concentration when the test substance is added after the enzymatic reaction (mg/dL)
B: glucose concentration when the test substance is added from the beginning (mg/dL)

[Example 4] Analysis of the effect of lotus leaf extract in suppressing blood glucose in sucrose-loaded mice

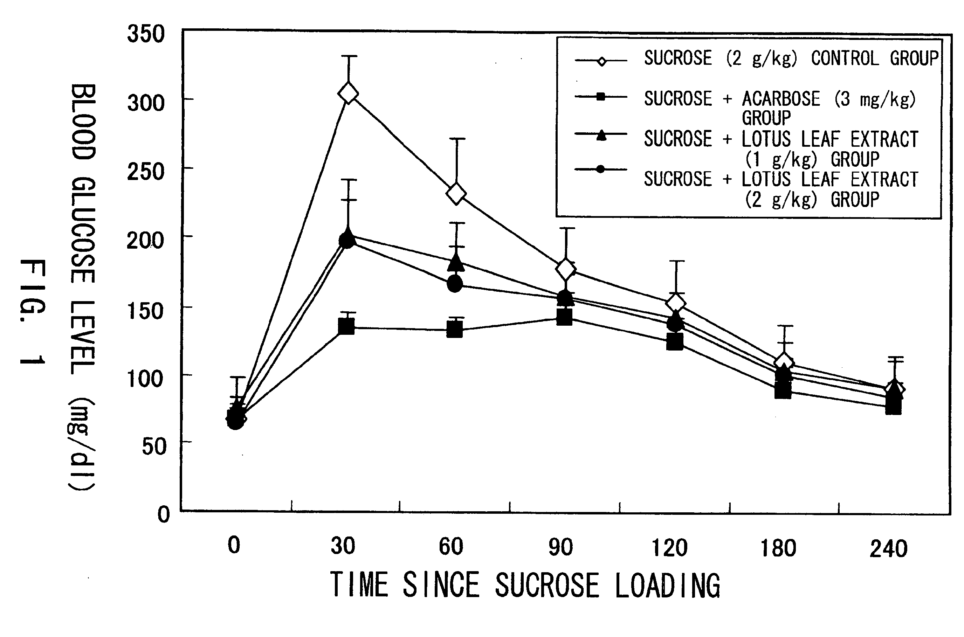

Five-week-old male ICR mice were purchased, and were prefatorily reared for one week. After prefatory rearing, the mice were separated into four groups of six animals each, according to weight. Lotus leaf extract and sucrose were simultaneously administered orally to the mice in two of these groups. The concentration of lotus leaf extract administered was 1 g/kg or 2 g/kg, and the sucrose concentration was 2 g/kg. As the control group, 3 mg/kg of acarbose was similarly orally administered to another group of mice at the same time as the sucrose. Sucrose alone was administered to the remaining group. The blood glucose levels of the mice in each group were measured. Blood glucose levels were measured immediately prior to oral administration of the test substance, and at 30, 60, 90, 120, 180, and 240 minutes after administration. The measurement results are shown in Fig. 1. The results revealed that the groups to which lotus plant extract was administered had suppressed increases in blood glucose levels compared to the group to which sucrose alone was administered. The suppressive effect on blood glucose increase was particularly large at 30 minutes after sucrose administration.

[Example 5] Analysis of the effect of dried lotus leaf extracts in improving obesity in high-fat diet-loaded obese mice

5-(1) Experimental materials and methods

The effects of administering dried lotus leaf extracts as test substances to high-fat-diet-loaded mice were examined. Six-week old female ICR mice were obtained from CLEA Japan, and were quarantined and conditioned for 14 days under rearing conditions the same as the test conditions. On the day of administration forty animals, which showed satisfactory weight gain during quarantine and conditioning with no abnormalities in their general condition, were used in the tests. The mice were grouped by stratified random sampling based on weight. The four test groups were: Group A (basic feed); Group B (high-fat diet); Group C (high-fat diet containing 2 % dried lotus leaf extract); and Group D (high-fat diet containing 5% dried lotus leaf extract). Each group consisted of ten animals. The mice were raised in two cages per group, with five mice of the same group housed in each cage. Powdered feed CE-2 (CLEA Japan) was used as the basic feed. The other feeds (the high-fat diet and high-fat diets containing dried lotus leaf extract) are described in Table 2. The values in the table show the amount of each ingredient to mix when preparing a total of one kilogram of feed mixture.

Table 2

| Preparation of the high-fat diets and the weighed amount of test substance | | | |
|---|---|---|---|
| | High-fat diet | High-fat diet containing 2% dried lotus leaf extract | High-fat diet tract containing 5% dried lotus leaf extract |
| CE-2 | 460g | 460g | 460g |
| Beef tallow | 400g | 400g | 400g |
| Granulated sugar | 90g | 90g | 90g |
| Corn starch | 50g | 30g | 0g |
| Dried lotus leaf extract | 0g | 20g | 50g |

In all groups, the feed was placed in a powder feeder, and the animals were free-fed. The water supply was tap water placed in a water supply bottle, which was free-fed through a nozzle. The test substance was continuously administered to the mice for ten weeks under the above-mentioned conditions. The general condition of the mice was observed, their body weight determined, the amount consumed was measured, and the mice were examined by autopsy, yielding the results below. The data shown below for body weight, feed intake, and organ weight are expressed as a mean $\pm$ S.D. Student's t-tests or Dunnett's multiple comparison tests were used to determine significant differences between the group on basic feed and the group on a high-fat diet; and between the group on a high-fat diet and the groups on the test substance.

5-(2) Observation of general condition

The general condition of every cage was observed once a day. Abnormalities were not observed in the general condition of any individual for the duration of the examination period.

5-(3) Body weight

An electronic top-loading balance was used to measure body weight immediately before administration of the test substance, and once a week after administration. The results for each group are individually shown in Table 3.

Body weight (g) of mice during high-fat diet tolerance test

| Group | Before test | After starting test (weeks) | | | | |
|---|---|---|---|---|---|---|
| | | 1 | 2 | 3 | 4 | 5 |
| A: Basic feed | 30.4 ±1.3 | 31.7 ±1.9 | 32.6 ±1.9 | 33.6 ±1.7 | 33.8 ±1.8 | 34.8 ±2.1 |
| B: High-fat diet | 30.5 ±1.3 | 32.7 ±1.4 | 34.7 ±1.2** | 35.6 ±2.1* | 37.5 ±2.6** | 39.2 ±3.4** |
| C: High-fat + 2% dried lotus diet          leaf extract | 30.5 ±1.3 | 32.2 ±2.2 | 34.9 ±2.7 | 36.1 ±3.0 | 36.9 ±3.0 | 37.7 ±3.2 |
| D: High-fat + 5% dried lotus diet          leaf extract | 30.5 ±1.3 | 31.0 ±1.4 | 33.4 ±1.7 | 34.5 ±2.3 | 35.8 ±2.7 | 35.5 ±1.9# |

| Group | After starting test (weeks) | | | | |
|---|---|---|---|---|---|
| | 6 | 7 | 8 | 9 | 10 |
| A: Basic feed | 35.2 ±2.4 | 35.6 ±2.9 | 36.6 ±2.8 | 38.9 ±3.5 | 39.3 ±3.0 |
| B: High-fat diet | 40.2 ±2.8** | 41.5 ±4.3** | 43.3 ±4.2** | 45.9 ±5.4** | 46.2 ±5.6** |
| C: High-fat + 2% dried lotus diet          leaf extract | 38.6 ±4.0 | 38.7 ±4.0 | 40.8 ±3.8 | 41.4 ±5.3 | 43.0 ±5.7 |
| D: High-fat + 5% dried lotus diet          leaf extract | 38.0 ±2.9 | 36.6 ±1.5## | 38.1 ±3.0## | 39.3 ±3.3## | 42.2 ±3.2 |

Each value represents mean ± S.D. (n=10)
*, P<0.05; **, P<0.01 (basic feed group vs. high-fat diet group: Student's t-test)
#, P<0.05; ##, P<0.01 (high-fat diet group vs. high-fat diet + dried lotus leaf extract group: Dunnett's test)

Group A (basic feed) and Group B (high-fat diet) were compared to examine the effect of a high-fat diet. The results showed that the body weight of mice in both groups increased steadily after starting the test, but that the body weight of mice in Group B increased more than in Group A. Significant differences were observed between the body weights of the two groups from two weeks after starting the test, up until completion of the test. At the end of the test, there was an average difference of 6.9 g between the two groups (Group A, 39.3 ± 3.0 g; Group B, 46.2 ± 5.6 g; p<0.01).

Group B, Group C (high-fat diet containing 2% dried lotus leaf extract), and Group D (high-fat diet containing 5 % dried lotus leaf extract) were compared to analyze the effect of the dried lotus leaf extract. After starting the test the body weights of all groups increased steadily, but the increase in body weight in Group C and Group D tended to be less than in Group B. Significant differences between Group D and Group B in particular were confirmed at 5, 7, 8, and 9 weeks after starting the test. Accordingly, dried lotus leaf extract was confirmed to have the effect of suppressing increases in body weight.

5-(4) Feed intake

Feed intake was measured twice a week, that is, every three or four days after test substance administration. The weight of the feed including the feeder was measured for each cage using an electronic top-loading balance, and intake was calculated by subtracting the weight of the remaining feed from the weight of the feed provided.

The average daily feed intake per animal, calculated for each cage, is shown in Table 4. For each cage, n=5; and for each group, n=10.

Table 4

**Average feed intake per animal for each cage**

Average feed intake (g/day)

| Group | Cage No. | First week First half | First week Second half | Second week First half | Second week Second half | Third week First half | Third week Second half | Fourth week First half | Fourth week Second half | Fifth week First half | Fifth week Second half |
|---|---|---|---|---|---|---|---|---|---|---|---|
| A: Basic feed group | 1 | 8.2 | 8.3 | 7.8 | 7.3 | 6.4 | 6.1 | 7.9 | 5.6 | 6.6 | 6.9 |
|  | 2 | 7.0 | 7.5 | 6.9 | 7.3 | 6.7 | 6.7 | 7.2 | 4.8 | 6.6 | 7.2 |
|  |  | 7.6 | 7.9 | 7.4 | 7.3 | 6.6 | 6.4 | 7.6 | 5.2 | 6.6 | 7.1 |
| B: High-fat diet group | 3 | 4.9 | 4.8 | 5.4 | 6.4 | 4.5 | 4.1 | 3.9 | 3.4 | 4.2 | 5.2 |
|  | 4 | 6.0 | 6.3 | 6.2 | 7.2 | 4.9 | 4.1 | 5.4 | 4.8 | 3.7 | 6.8 |
|  |  | 5.5 | 5.6 | 5.8 | 6.8 | 4.7 | 4.1 | 4.7 | 4.1 | 4.0 | 6.0 |
| C: High-fat diet + 2% dried lotus leaf extract group | 5 | 6.5 | 4.5 | 5.7 | 7.1 | 4.7 | 3.6 | 4.7 | 3.2 | 2.9 | 3.3 |
|  | 6 | 4.5 | 3.7 | 5.3 | 5.1 | 3.8 | 3.7 | 4.9 | 3.3 | 4.1 | 3.5 |
|  |  | 5.5 | 4.1 | 5.5 | 6.1 | 4.3 | 3.7 | 4.8 | 3.3 | 3.5 | 3.4 |
| D: High-fat diet + 5% dried lotus leaf extract group | 7 | 3.4 | 3.2 | 5.5 | 3.9 | 4.3 | 4.1 | 4.1 | 4.0 | 3.0 | 3.2 |
|  | 8 | 3.7 | 3.2 | 5.7 | 5.8 | 4.3 | 3.8 | 6.6 | 3.3 | 3.4 | 3.9 |
|  |  | 3.6 | 3.2 | 5.6 | 4.9 | 4.3 | 4.0 | 5.4 | 3.7 | 3.2 | 3.6 |

Average feed intake (g/day)

| Group | Cage No. | Sixth week First half | Sixth week Second half | Seventh week First half | Seventh week Second half | Eighth week First half | Eighth week Second half | Ninth week First half | Ninth week Second half | Tenth week First half | Tenth week Second half |
|---|---|---|---|---|---|---|---|---|---|---|---|
| A: Basic feed group | 1 | 6.0 | 6.1 | 6.4 | 6.2 | 7.6 | 6.2 | 6.2 | 7.0 | 5.3 | 6.0 |
|  | 2 | 6.5 | 6.0 | 4.9 | 5.1 | 7.1 | 5.1 | 4.9 | 4.3 | 5.2 | 6.2 |
|  |  | 6.3 | 6.1 | 5.7 | 5.7 | 7.4 | 5.7 | 5.6 | 5.7 | 5.3 | 6.1 |
| B: High-fat diet group | 3 | 3.1 | 3.7 | 3.5 | 3.5 | 3.7 | 3.4 | 3.6 | 3.4 | 3.5 | 3.3 |
|  | 4 | 3.3 | 5.6 | 4.0 | 2.7 | 3.2 | 2.9 | 3.7 | 2.6 | 4.1 | 2.9 |
|  |  | 3.2 | 4.7 | 3.8 | 3.1 | 3.5 | 3.2 | 3.7 | 3.0 | 3.8 | 3.1 |
| C: High-fat diet + 2% dried lotus leaf extract group | 5 | 2.9 | 2.8 | 2.9 | 2.7 | 3.0 | 2.7 | 3.0 | 3.0 | 3.1 | 2.9 |
|  | 6 | 3.1 | 4.0 | 3.1 | 3.0 | 3.8 | 3.1 | 2.4 | 2.7 | 2.7 | 3.1 |
|  |  | 3.0 | 3.4 | 3.0 | 2.9 | 3.4 | 2.9 | 2.7 | 2.9 | 2.9 | 3.0 |
| D: High-fat diet + 5% dried lotus leaf extract group | 7 | 3.8 | 3.6 | 3.4 | 2.6 | 3.2 | 3.0 | 3.4 | 3.2 | 3.1 | 4.2 |
|  | 8 | 3.4 | 3.7 | 3.7 | 4.7 | 2.9 | 3.3 | 3.1 | 3.3 | 3.0 | 3.4 |
|  |  | 3.6 | 3.7 | 3.6 | 3.7 | 3.1 | 3.2 | 3.3 | 3.3 | 3.1 | 3.8 |

The average daily feed intake per animal was 5.2 to 7.9 g/day in Group A, 3.0 to 6.8 g/day in Group B, 2.7 to 6.1 g/day in Group C, and 3.1 to 5.6 g/day in Group D. The intake for Group A was clearly greater than in the other groups. Although feed intake tended to be greater in the first half of the test (around the second week) and less in the latter half of the test (sixth week and beyond), there was no particular change in each of Groups B to D.

5-(5) Organ weight

At the end of the observation period (ten weeks after starting the test), the mice were autopsied and their organ weights (wet weights of the liver, kidneys, and fat) were measured. The results are shown in Table 5. In each group, n=10.

Mouse organ weight on completion of the high-fat diet tolerance test

Table 5

EP 1 690 519 A1

| Group | Number | Total liver weight (g) | Kidney weight (g) | Fat weight (g) |
|---|---|---|---|---|
| A: Basic feed | 10 | 1.9332 ± 0.2201 | 0.4164 ± 0.0387 | 1.5620 ± 0.6598 |
| B: High-fat diet | 10 | 2.0428 ± 0.3107 | 0.3680 ± 0.0368* | 3.3871 ± 1.3395** |
| C: High-fat + 2% dried diet lotus leaf extract | 10 | 1.9430 ± 0.2213 | 0.3463 ± 0.0312 | 2.6391 ± 1.0912 |
| D: High-fat + 5% dried diet lotus leaf extract | 10 | 2.0869 ± 0.2408 | 0.3934 ± 0.0358 | 1.7292 ± 0.5215## |

Each value represents mean ± S.D.
*, P<0.05; **, P<0.01 (basic feed group vs. high-fat diet group: Student' s t-test or Aspin-Walch's t-test)
#, P<0.05; ##, P<0.01 (high-fat diet group vs. high-fat diet + dried lotus leaf extract group: Dunnett's test)

When Groups A and B were compared to analyze the effect of the high-fat diet, no significant difference was observed for the liver weight. However, in Group B the kidneys were significantly lighter, and the fat was significantly heavier.

When Groups B, C, and D were compared to analyze the effect of the dried lotus leaf extract, the liver and kidney weights did not show significant differences, but the fat weight was low in both Groups C and D. Significant differences were observed between Group D and Group B in particular, confirming the effect of the dried lotus leaf extract in reducing fat.

5-(6) Summary

As described above, although the group taking 2% dried lotus leaf extract did display a trend towards suppressed increase in body weight, the effect was not significant. On the other hand, significant suppression of body weight increase was observed in the group taking 5% dried lotus leaf extract. However, since there was some difference in feed intake, further examination was required to determine whether the weight loss was due to the level of feed intake or to the effect of the dried lotus leaf extract. Accordingly, the amount of feed necessary to increase body weight by 1 g was calculated for each group, using total feed intake during the test and the amount of weight increase at completion of the test. This amount was 19.1 g for the group on a high-fat diet, 20.2 g for the group taking 2% dried lotus leaf extract (high-fat diet + 2 % dried lotus leaf extract), and 22.7 g for the group taking 5% dried lotus leaf extract (high-fat diet + 5% dried lotus leaf extract). This finding confirmed a dose-dependent increase. Therefore, it can be concluded that the suppression of weight increase observed in this Example was a result of the dried lotus leaf extract. The weight of fat at the time of autopsy tended to be reduced in the group taking 2% dried lotus leaf extract, and was significantly reduced in the group taking 5% dried lotus leaf extract, and these results support the above conclusion.

[Example 6] Effect of dried lotus leaf extract on carbohydrate metabolism and lipid metabolism in humans

6-(1) Experimental materials and methods

An objective of this Example was to analyze the effect of the dried lotus leaf extract on carbohydrate metabolism and lipid metabolism in humans. To carry out analyses with the above aim, human subjects were limited to those who satisfied a given set of conditions. A subject in this Example must satisfy the following conditions:

1) They must be a so-called borderline-type subject. Specifically, the subject's fasting blood glucose level must be 110 to 126 mg/dl, or their blood glucose level two hours after taking a 75 g glucose tolerance test (OGTT) must be 140 to 200 mg/dl. Individuals whose fasting blood glucose level is 126 mg/dl or more, or whose blood glucose level at two hours after 75 g OGTT is 200 mg/dl or more, were defined as diabetes-type subjects and were therefore excluded from the subjects of this Example.

2) They must have a Body Mass Index (BMI) of 22 or more. BMI is also called physique index and is a value determined by dividing a subject's body weight (kg) by the square of their height (m). BMI = body weight (kg) / height $(m)^2$

3) They must not have received medication for diabetes.

4) They must not have a severe liver, kidney or cardiovascular disorder, or a food allergy.

5) They must be 40 to 58 years old if male, and 40 to 55 years old if female, and their daily life must be similar to that of a healthy individual.

6) Their daily intake of tea must be 2 L or less.

Questionnaires and such were given to those individuals who satisfied the above six conditions, and subjects were selected. The questionnaire and interview asked the following: A) daily calorie intake; B) food preference; and C) medical history, family history, work and living environment, exercise habits, and smoking and drinking habits.

95 individuals who matched the subject conditions were selected as subjects. The selected subjects were divided into three groups such that there was no difference between the groups in terms of age, sex, and fasting blood glucose level. Two of the groups took dried lotus leaf extract (Group T and Group R) and the other took a placebo (Group S). As the control group, individuals in Group S consumed a basic tea (200 mL/bottle) that did not comprise the lotus leaf extract. As the test substance-taking groups, individuals in Group T consumed a tea comprising 0.5 g/200 mL of dried lotus leaf extract powder, and individuals in Group R consumed a tea comprising 1.0 g/200 mL of dried lotus leaf extract powder. The constitution and average age of each group are shown in Table 6. Group S (placebo): n=30; Group T (1 g of dried lotus leaf extract/day): n=34; and Group R (2 g of dried lotus leaf extract/day): n=31.

Table 6

| Group constitution and average age in human test groups | |
|---|---|
| Group constitution | Average age ± S.D. (number of subjects) |
| S (placebo) | 48.3±4.7(n=30) |
| T (1 g of dried lotus leaf extract/day) | 50.6±5.0(n=34) |
| R (2 g of dried lotus leaf extract/day) | 48.9±4.6(n=31) |

The test was carried out using a double-blind test with the placebo group as a control. The subjects were observed for two weeks prior to intake of the test food, and then consumed two bottles of the above-mentioned tea per day (200 mL x 2/day), one bottle in the morning and another in the afternoon, for 12 weeks. Physical measurements and sugar tolerance tests were carried out on the subjects to analyze the effect of the dried lotus leaf extract.

6-(2) Physical measurements

Physical measurements were taken for each subject before intake, and six and 12 weeks after intake. The measured characteristics were height, weight, waist circumference, BMI, hip circumference, percent body fat, and level of visceral fat. Percent body fat and level of visceral fat were measured using an Omron Body Composition Monitor HBF-352. A visceral fat level of 10 corresponds to visceral fat area of 100 cm$^2$. The results are shown in Tables 7 and 8. For both Tables 7 and 8, Group S (placebo): n=30; Group T (1 g of dried lotus leaf extract/day): n=34; and Group R (2 g of dried lotus leaf extract/day): n=31.

Table 7

Human tests (Physique Index 1)

| | | | | | | Mean ± S. D. |
|---|---|---|---|---|---|---|
| | Group | Pre-test value | Value at six weeks | Value at twelve weeks | Change (at six weeks) | Change (at twelve weeks) |
| Body weight (kg) | S | 71.7±10.0 | 71.0±9.9 | 70.5±10.1 | -0.7±0.9 | -1.2±1.5 |
| | T | 71.5±11.3 | 70.2±11.0 | 69.2±10.9 | -1.3±1.1 | -2.3±1.6# |
| | R | 71.5±9.7 | 69.7±9.9 | 68.9±10.1 | -1.8±1.1* | -2.7±1.4* |
| BMI | S | 25.6±2.4 | 25.3±2.4 | 25.1±2.4 | - 0.3±0.3 | -0.5±0.5 |
| | T | 25.7±2.8 | 25.3±2.8 | 24.9±2.7 | -0.4±0.4 | -0.8±0.5# |
| | R | 25.6±2.5 | 24.9±2.6 | 24.6±2.6 | -0.7±0.5* | -1.0±0.6* |
| Percent body fat (%) | S | 28.6±5.2 | 28.1±5.5 | 27.0±5.7 | -0.6±1.1 | -1.6±1.3 |
| | T | 29.3±4.7 | 27.7±4.5 | 26.7±4.4 | -1.5±1.3# | -2.6±1.9# |
| | R | 28.8±4.6 | 27.0±4.5 | 25.7±5.1 | -1.7±1.0* | -3.0±1.6* |

[S vs T]p<0.05 : # [S vs R] P<0.05: * (Dunnett's test)

Table 8

Human tests (Phys i que Index 2)

| | | | | | | Mean ± S. D. |
|---|---|---|---|---|---|---|
| | Group | Pre-test value | Value at six weeks | Value at twelve weeks | Change (at six weeks) | Change (at twelve weeks) |
| Visceral fat level | S | 10.4±3.3 | 10.1±3.3 | 9.8±3.3 | -0.3±0.7 | -0.6±0.7 |
| | T | 11.4±4.0 | 10.9±4.0 | 10.3±3.9 | -0.5±0.7 | -1.1±1.2# |
| | R | 11.3±3.9 | 10.3±4.0 | 9.8±3.9 | -0.9±0.6* | -1.4±0.7* |

(continued)

| Human tests (Phys i que Index 2) | | | | | | |
|---|---|---|---|---|---|---|
| | | | | | | Mean $\pm$ S. D. |
| | Group | Pre-test value | Value at six weeks | Value at twelve weeks | Change (at six weeks) | Change (at twelve weeks) |
| Waist circumference (cm) | S | 86.9$\pm$7.3 | 86.5$\pm$7.3 | 86.3$\pm$7.2 | -0.4$\pm$0.6 | -0.6$\pm$1.1 |
| | T | 87.1$\pm$8.6 | 85.9$\pm$8.5 | 84.9$\pm$8.2 | -1.2$\pm$1.4[#] | -2.2$\pm$1.7[#] |
| | R | 87.2$\pm$8.0 | 85.3$\pm$7.9 | 84.8$\pm$7.9 | -1.9$\pm$1.2[*] | -2.4$\pm$1.7[*] |
| Hip circumference (cm) | S | 97.9$\pm$6.3 | 97.5$\pm$6.1 | 97.3$\pm$6.0 | -0.3$\pm$1.1 | -0.5$\pm$1.4 |
| | T | 96.7$\pm$6.9 | 96.4$\pm$6.7 | 95.7$\pm$6.6 | -0.3$\pm$0.8 | -1.0$\pm$2.0[#] |
| | R | 97.9$\pm$6.4 | 96.5$\pm$5.7 | 96.3$\pm$5.8 | -1.4$\pm$1.8 | -1.6$\pm$2.1[*] |

[S vs T] $p<0.05$: # [S vs R] $p<0.05$: * (Dunnett's test)

Compared to subjects in the control group, subjects taking 2 g/day of dried lotus leaf extract for six and 12 weeks showed significantly lowered body weight, BMI, percent body fat, visceral fat level, waist circumference, and hip circumference, showing the effect of dried lotus leaf extract in reducing such values. Taking 1 g/day of dried lotus leaf extract for six weeks also significantly lowered body weight and percent body fat compared to the control group. Intake for 12 weeks significantly lowered body weight, BMI, percent body fat, visceral fat level, and waist circumference compared to the control group.

6-(3) Glucose tolerance tests

Glucose tolerance tests (75 g glucose/body) were conducted twice for each subject: once before intake and once 12 weeks after intake. The subjects were not allowed to eat or drink after 9 p.m. the night before blood collection. On the day of testing, blood was first collected under fasting conditions and taken to be blood before glucose loading. Next, a 75 g glucose load was given, and blood was collected over time, that is, at 30, 60, and 90 minutes after loading.. The test was completed by 11 a.m. Blood glucose level in the plasma was measured using a Hitachi automatic analyzer 7075.

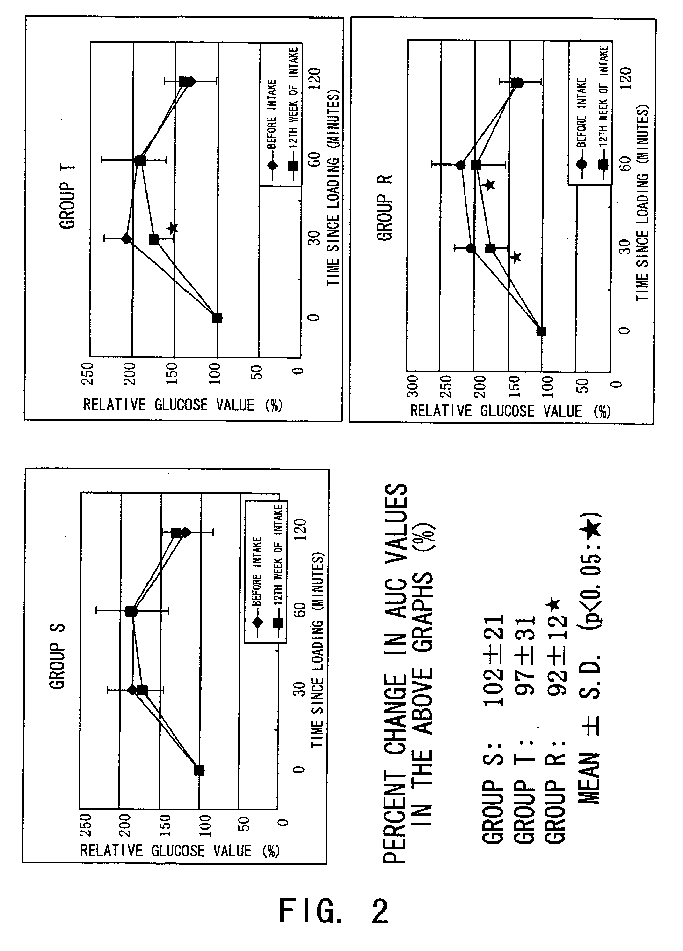

The results are shown in Fig. 2. In Fig. 2, the blood glucose level at each time point is expressed as a relative glucose level (%) in comparison to the blood glucose level before the glucose load is given. Furthermore, based on the relative glucose levels at each time point, the area under the curve (AUC) was calculated for 0 to 120 minutes. Group S (placebo): n=30; Group T (1 g of dried lotus leaf extract/day): n=34; and Group R (2 g of dried lotus leaf extract/day): n=31. Intake of dried lotus leaf extract for 12 weeks was shown to suppress blood glucose increase at 30 minutes and 60 minutes after glucose loading.

6-(4) Summary

As described above, as for the animal tests, dried lotus leaf extract was found to have the effect of reducing body fat in humans. The reduction in body fat due to a daily intake of 2 g of dried lotus leaf extract/day for six weeks was reflected as a reduction in weight in humans. A similar effect was also confirmed for an intake of 1 g/day for 12 weeks. Such effects and the results of glucose tolerance tests suggest that the lotus leaf extracts have the effect of reducing body fat as well as reducing insulin resistance.

Industrial Applicability

α-glucosidase activity inhibitors comprising *Nelumbonaceae* plant preparations enabled suppression of increases in blood glucose. Accordingly, new options are provided for therapeutic or preventive pharmaceuticals for diabetes, a disease for which patients are increasing.

**Claims**

1.   An α-glucosidase activity inhibitor comprising a *Nelumbonaceae* plant preparation.

**2.** A pharmaceutical composition for treating or preventing diabetes, wherein the composition comprises the α-glucosidase activity inhibitor of claim 1.

**3.** A food composition for treating or preventing diabetes, wherein the composition comprises the α-glucosidase activity inhibitor of claim 1.

SUCROSE (2 g/kg) CONTROL GROUP

SUCROSE + ACARBOSE (3 mg/kg) GROUP

SUCROSE + LOTUS LEAF EXTRACT (1 g/kg) GROUP

SUCROSE + LOTUS LEAF EXTRACT (2 g/kg) GROUP

BLOOD GLUCOSE LEVEL (mg/dl)

TIME SINCE SUCROSE LOADING

FIG. 1

FIG. 2

## INTERNATIONAL SEARCH REPORT

| | International application No. |
|---|---|
| | PCT/JP2004/016333 |

**A. CLASSIFICATION OF SUBJECT MATTER**
Int.Cl⁷ A61K35/78, A61P3/10, 43/00, A23L1/30, C12N9/99

According to International Patent Classification (IPC) or to both national classification and IPC

**B. FIELDS SEARCHED**

Minimum documentation searched (classification system followed by classification symbols)
Int.Cl⁷ A61K35/78, A61P3/10, 43/00, A23L1/30, C12N9/99

Documentation searched other than minimum documentation to the extent that such documents are included in the fields searched

Electronic data base consulted during the international search (name of data base and, where practicable, search terms used)
JICST(JOIS)

**C. DOCUMENTS CONSIDERED TO BE RELEVANT**

| Category* | Citation of document, with indication, where appropriate, of the relevant passages | Relevant to claim No. |
|---|---|---|
| P,X | JP 2004-168770 A (Ikeda Shokken Kabushiki Kaisha), 17 June, 2004 (17.06.04), Table 1 (Family: none) | 1-3 |
| X | JP 2003-128571 A (Matsuura Yakugyo Kabushiki Kaisha), 08 May, 2003 (08.05.03), Page 4; table 1 (Family: none) | 1-3 |
| X | Yukitaka FUKAYA et al., "Himan Taisaku Sozai Toshiteno Kayo (Kayo Extract no Kohiman Koka)", New Food Industry, 01 May, 2003 (01.05.03), Vol.45, No.5, pages 41 to 48 | 1-3 |

☐ Further documents are listed in the continuation of Box C.  ☐ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 25 November, 2004 (25.11.04) | Date of mailing of the international search report 14 December, 2004 (14.12.04) |
|---|---|
| Name and mailing address of the ISA/ Japanese Patent Office | Authorized officer |
| Facsimile No. | Telephone No. |

Form PCT/ISA/210 (second sheet) (January 2004)

19